# EUROPEAN PATENT APPLICATION

(11) **EP 2 039 254 A1**
(43) Date of publication of application: **25.03.2009**
(21) Application number: 07116508.8
(22) Date of filing: 14.09.2007
(51) Int. Cl.: A23L 1/00, A23L 1/22, A61K 9/51, A61K 8/19, A61Q 11/00, A61K 8/73, A61K 8/64, A01N 25/10

(54) **Hybrid inducible release vehicle**

(71) Applicant: Nederlandse Organisatie voor Toegepast- Natuurwetenschappelijk Onderzoek TNO, 2628 VK Delft (NL)
(72) Inventor: Slaghek, Theodoor Maximiliaan, 3042 HT, Rotterdam (NL); Timmermans, Johannes Wilhelmus, 6714 GG, Ede (NL); Batenburg, Lawrence Fabian, 5652 EJ, Eindhoven (NL); van Ee, Renz Jeroen, 3991 XV, Houten (NL)
(74) Representative: Hatzmann, Martin

(57) **Abstract**

The invention is directed to a hybrid inducible release vehicle, to a method for preparing such a vehicle, and to the use thereof.
The hybrid inducible release vehicle of the invention comprises a gel, which gel comprises a cross-linked carbohydrate or protein polymer and an inorganic carrier, and at least one active ingredient bound to said inorganic carrier, wherein the release of the active ingredient is triggered by contact of the vehicle with an external stimulus.

## Description

The invention is directed to a hybrid inducible release vehicle, to a method for preparing such a vehicle, and to the use thereof. This type of vehicles is particularly usable for packaging an active component in applications in which said active component needs to be shielded from the environment until it is at a time and/or place where it should be released. One of the major applications lies in the field of antimicrobial active components, which need only to be released in the presence of microbial contamination. This can, for instance, be the case in antimicrobial packages, which have as their general object to prolong the storage life of the packaged foods by preventing decay by micro-organisms.

WO-A-95/17816 describes release vehicles in which an active ingredient is continuously released, also when there is no demand. GB-A-2 198 062 describes induced release vehicles in which an active ingredient is released under influence of mechanical activity. However, since mechanical activity is required for the release, the applications for such an induced release vehicle are rather limited.

Degradable release vehicles are disclosed in WO-A-99/08553, wherein the vehicles are made of "edible polymers" such as polyvinylpyrrolidone, polyethylene wax, etc. A special form of degradable vehicles is described in WO-A-95/33773 in which capsules of chitine or chitosan are presented containing an active ingredient. These vehicles would be degradable by lysozyme through hydrolysis. GB-A-1 576 999 describes the use of biopolymers which are coagulated at elevated temperature (120°C) in "vasiline petroleum gelly" and contain either organic tin compounds or Cu₂O. These particles are used as additive in anti fouling paint. The disadvantage of this system is that for instance heat sensitive and/or organic solvent sensitive active ingredients cannot be used and also the formed vehicles cannot be filled with an active ingredient once the vehicle is formed.

WO-A-2004/105485 describes an inducible release vehicle based comprising a charged cross-linked carbohydrate or a protein polymer and an active component. The release is triggered by contact of the vehicle with an external stimulus, such as a change in pH or a change in salt concentration. However, the inventors found that active ingredients with no or only few charges, as well as active ingredients with hydrophobic groups are not satisfactorily bound within the vehicle. Furthermore, it was found that the loading level for specific active ingredients was not satisfactory.

Object of the invention is therefore to provide release vehicles that are able to encapsulate an active ingredient and to release the active ingredient on demand, *i*.*e*. at a specified place and/or time, due to an external (physical, chemical or enzymatical) trigger or stimulus, which at least partly overcome the above-mentioned disadvantages.

This object was met by providing hybrid inducible release vehicles comprising both organic and inorganic components.

Accordingly, in a first aspect the invention is directed to hybrid inducible release vehicle for releasing an active ingredient, comprising a gel, which gel comprises a cross-linked carbohydrate or protein polymer and an inorganic carrier, and at least one active ingredient bound to said inorganic carrier, wherein the release of the active ingredient is triggered by contact of the vehicle with an external stimulus.

The inventors found that such hybrid inducible release vehicles allow a higher loading of active components and a wider range of active ingredients which can be released than the release vehicles of the prior art.

Another advantage is that the sensitivity of the hybrid release vehicles to external conditions is reduced with respect to the vehicles described in WO-A-2004/105485 in that the active ingredients in the hybrid release vehicles are not released when subjected to a relatively large salt concentration.

In addition, the charge density and charge distribution in the hybrid release vehicles of the invention is very high and the polarity of the gel of the hybrid release vehicles can be reversed. The advantage of having a high charge density in the hybrid release vehicles is that more acceptor sites for active ingredients are created. Further, the type of charge (positive or negative) will be controlled by the inorganic carrier and will not depend on the charge present on the polymer backbone. This allows for a more flexible delivery system then described in the prior art.

The hybrid inducible release vehicles of the invention comprise a gel which comprises a cross-linked carbohydrate or protein polymer and an inorganic carrier.

The cross-linked carbohydrate or protein polymer, can be made of oligomeric and polymeric carbohydrates or proteins which can be used as a substrate for any external stimulus, such as an enzyme. Carbohydrates which can thus be used are carbohydrates consisting only of C, H and O atoms such as glucose, fructose, sucrose, maltose, arabinose, mannose, galactose, lactose and oligomers and polymers of these sugars, cellulose, dextrins such as maltodextrin, agarose, amylose, amylopectin and gums, *e*.*g*. guar. Proteins which can be used include albumin, ovalbumin, casein, myosin, actin, globulin, hemin, hemoglobin, myoglobin and small peptides. Preferably, oligomeric carbohydrates from DP2 on or polymeric carbohydrates from DP50 on are used. These can be naturally occurring polymers such as starch (amylose, amylopectin), cellulose and gums or derivates hereof which can be formed by phosphorylation or oxidation. Other polymers can also be used (*e*.*g*. caprolactone), which can be added for a better compatibility with *e*.*g*. the packaging material. In the case of proteins, proteins obtained from hydrolysates of vegetable or animal material can also be used. Also suitable mixtures of carbohydrates (*e*.*g*. copolymers) or mixtures of proteins can be used.

The advantages of cross-linked polymers lies in the intrinsic stability of the vehicles formed through the introduction of cross links in the matrix. Specifically, the cross-links are ether- and/or ester-links, where for the ester-links phosphate-esters are preferable. A further important advantage is that cross-linking provides a three-dimensional lattice of the polymer, in which the inorganic carrier and the active component can be encapsulated. Moreover, the choice of components, *i*.*e*. the choice of polymer(s) and cross-linker(s) influence the three-dimensional structure of the vehicle and thus allow for the manufacture of specific vehicles suited for molecules of a certain size and/or certain charge.

The polymer matrix from which the vehicle is built may be constructed from readily available and water soluble polymers such as polysaccharides and (hydrolysed) proteins and in doing so a flexible matrix may be formed to generate a custom made vehicle.

Optionally, the polymer matrix can be positively and/or negatively charge through *e*.*g*. carboxylic acids and/or cationic groups. Such charges can be provided by the polymer itself, but can also be introduced as a result of modification of the polymer or by the cross-linker used for cross-linking the polymer.

The formation of the matrix is accomplished through covalent cross-linking of the polymers. Typical cross-linkers, that can be used, are divinyl sulphone, epichlorohydrin, a di-epoxide such as glycerol diglycidyl ether or butanedioldiglycidyl ether, sodium trimetaphosphate and adipic acid or derivatives thereof, and the like. Cross-linking can also be established by enzymatic action, *e*.*g*. by using enzymes from the group consisting of laccases (which *e*.*g*. induce cross-linking of pectins), peroxidases, polyphenol oxidases, transglutaminases, protein disulfide isomerases, sulfhydryl oxidases, lysyl oxidases and lipoxygenases. Methods how to use these cross-linkers or cross-linking enzymes are well known in the art and/or have been abundantly described in the experimental part.

The polymers may be modified by oxidation, substitution with cationic functional groups or carboxymethyl groups and/or esterifying with *e*.*g*. acetyl groups. Such modification generally is carrier out before cross-linking and gel formation. Only if cross-linking by ether-forming has been done it is possible to modify the polymer after cross-linking and gel formation. The person skilled in the art will know how to modify the polymers specified in the invention to provide them with the mentioned groups.

Advantageously, the polymers are of considerable size, i.e. 30 kD or more. This allows for the ready formation of a gel upon cross-linking and it allows for the formation of a lattice which is capable of taking up the inorganic carrier and the active component.

The inorganic carrier is used to bind the active ingredient in the vehicle, preferably through specific binding sites on the inorganic carrier. This has the advantages that the polymer matrix does not necessarily have to be charged and that the loading of the vehicle is increased.

The inorganic carrier can be inserted into the polymer matrix after gel formation, but it is also possible to carry out an *in situ* synthesis of the inorganic carrier, i.e. components of which the inorganic carrier is comprised are inserted in the polymer matrix and the inorganic carrier is synthesised inside the polymer matrix.

Preferably, the inorganic carrier is in the form of particles. It is preferred that the particles are limited in size so that they can be readily introduced into the polymer matrix. Preferred average particle sizes as measured by transmission electron microscopy are 2-1 000 nm, preferably 5-500 nm, more preferably 10-100 nm. The shape of the particles can vary. The particles may for instance have a spherical shape, a needle shape, or a platelet shape. The aspect ratio (the ratio between the length and the thickness of the particles) can be 1-500.

Examples of preferred inorganic carriers include particles of Ag, Au, Fe₂O₃, MgCl₂, AlCl₃, ZnCl₂, SiO₂, TiO₂, ZnO, MgO, smectites, or layered double hydroxides. The latter are in particular preferred. It is further advantageous that the inorganic carrier, in addition to the active component, can possess an advantageous property, such as an antimicrobial property, an UV blocking property, a magnetic property, improved absorption properties, or a property related to food and/or pharma additives.

The inorganic carrier may be charged, either negative or positive. Such charges may assist in binding the active component. However, it is also possible that the inorganic carrier is neutral and that the active component binds at specific sites, such as adsorption or metal-ligand sites. The binding may further involve hydrogen bonding.

The active ingredient can for instance be introduced into the vehicle by extraction or ion exchange. It is also possible to pre-load the inorganic carrier with the active ingredient and then to subsequently introduce the complex into the vehicle.

The active component can be of any size and weight, but in view of the insertion into the vehicle it preferably has a weight of less than 50 kD, more preferably less than 30 kD and most preferably less than 10 kD.

The active component which is available in the vehicle will not be released unless an external stimulus changes the property of the vehicle. This has the advantage that the active component is not spilled to the environment or - in case of pharmaceutical and/or nutraceutical carriers - to parts of the body where it is not wanted, or even toxic. The stimulus can be of any origin, as long as it is able to open up the vehicle or reduce the binding of the active ingredient with the vehicle so that the active component will be released. The active ingredient can thus be released for instance by a change in the pH, a change in salt concentration of the environment, and/or hydrolysis of the polymer. Also a change in water concentration, a change in temperature and the presence of enzymes and/or bacteria which case degradation of the polymer are suitable external stimuli to release the active ingredient.

In one embodiment, the active ingredient is also released from the inorganic carrier by the external stimulus. A change in the pH, a change in salt concentration, and a change of temperature are particularly suitable external stimuli for releasing the active ingredient from the inorganic carrier.

It is also possible that the inorganic particle dissolves upon degradation of the polymer, thereby releasing the active ingredient. This can for instance be the case if inorganic particles that are soluble in acid (such as MgCl₂, AlCl₃ or ZnCl₂) are released in an acid environment.

It is, however, not required that the active ingredient is released from the inorganic carrier. The complex of active ingredient with inorganic carrier can itself be active, so that only the complex of active ingredient with inorganic carrier needs to be released from the polymer. For instance, upon degradation of the polymer a silicate comprising the active ingredient may be released in acid environment, wherein the active compound can exert its activity while it is not fully dissolved.

In a preferable embodiment, the invention encompasses vehicles in which the external stimulus is an enzyme which is able to degrade the polymer. A large number of enzymes which can convert the above-mentioned polymers whereupon the embedded active component is released, are known, such as amylase, hemicellulase, xylanase, glucanase, pullulanase, arabinodase, cellulase, pectinase, mannanase or peptidase or protease.

The active ingredient can for instance be an antimicrobial substance, preferably bacteriocins, such as nisin and pediocin; metals or derived metals, such as metal oxides, metal salts, metal complexes or alloys; antibiotics, such as penicillin, erythromycin, ampicillin, isoniazid, tetracycline, sulphonamides and chloramphenicol; vegetable toxins, such as defensins, lectins, and anti fungal proteins; ethanol; H₂O₂-producing enzymes such as oxidases; organic acids such as propionic acid and derived propionates, sorbic acid and derived sorbates, benzoic acid and derived benzoates, lactic acid; sodium diacetate; sodium nitrite; lysozyms and antimicrobial substances from spices.

In embodiments wherein the encapsulated antimicrobial active ingredient is administered to the human body, which is preferably done through the oral route, preferably antimicrobial substances are used which are qualified as "foodgrade" by the food and drug administration. Such antimicrobial substances can, for instance, be obtained from herbs and/or spices. Antimicrobial substances (*e*.*g*. defensins) produced by plants for defence against bacterial or fungous infections are also usable. Finally, mention should be made of the category of antimicrobial substances produced by fungi which are already being incorporated into the food (*e*.*g*. in the preparation of cheese).

Alternatively, active components can be chosen from various groups of components such as pharmaceutical and/or nutraceutically active compounds, odorants, flavouring compounds, seasoning compounds, etc. Pharmaceutical and/or nutraceutical active compounds are preferably chosen from compounds which need to be administered in the close vicinity of the cell or organ where they should have their pharmaceutical and/or nutraceutical effect, and which, when given systemically would yield unwanted or even toxic side effects. One group of such compounds are antibiotic compounds, such as the above discussed antimicrobials, where the application needs to be local, *e*.*g*. in the mouth (anti-caries). Another group of active ingredients are cytostatic compounds, for use in anti-cancer medication. The external stimulus which could trigger the release of compounds in this case, could for instance be the pH, since it is known that the pH in tumours is lower than in the rest of the body (about 6.8-7.0 in tumours and 7.2-7.3 in blood). It lies well within the skill of the person in the art to produce vehicles which would remain stable at normal body pH, but which would be starting the release active components (in this case the cytostatic compound) if a lower pH is encountered.

Anadvantage of the delivery of pharmaceutical and/or nutraceutically active components through the vehicles of the invention is that the active compound is preserved by the vehicle and will not be metabolised and/or degraded in the body.

Especially, the advantage of the present invention is that delivery of nutraceutically active ingredients through the use of vehicles results in passage through the stomach of acid labile components in an intact form, which are released in *e*.*g*. the large intestine through the action of enzymes released by the intestinal flora.

It is also possible, according to the present invention, to provide two or more active components. This can be achieved by mixing vehicles loaded with different components or by providing a loading solution with two or more active components solved therein for loading the vehicles.

In a further aspect the invention is directed to a method for preparing the hybrid inducible release vehicle of the present invention. Such preparation method comprises
- providing a mixture of the polymer and a cross-linking agent;
- cross-linking the polymer thereby forming a gel;
- adding the inorganic carrier;
- adding the gel with the at least one active ingredient.

The polymer can be solved in a mixture of water and base before the cross-linking agent is added. After addition of the cross-linker, the gel is formed, typically within 1-24 hours. Thereafter, the inorganic carrier, or precursors thereof, can be added to a solution of the gel. Precursors can for instance include precursors for layered double hydroxides, such as Mg(NO₃)₂·6H₂O and Al(NO₃)₃·9H₂O. Preferably the pH is kept within the range of 7-10, more preferably within the range of 7-8. The gel can then be isolated by decanting, filtration and drying.

The amount of inorganic carrier in the vehicle can be at least 0.5 wt.% based on dry weight solids, preferably at least 2 wt.%, more preferably at least 4 wt.%. The upper limit of inorganic carrier in the vehicle may be 99 wt.% based on dry weight solids, preferably 80 wt.%, and more preferably 40 wt.%.

The gel provided with the inorganic carrier can thereafter be loaded with active ingredient by mixing the active ingredient with a solution of the gel under appropriate conditions depending on the inorganic carrier used and the nature of the applied active ingredient.

According to the method of the invention, the reactants may also be provided in a different sequence. It is for instance also possible to first provide the inorganic carrier and the active ingredient and subsequently add this composition to a solution of the gel in order to form the hybrid inducible release vehicle. In another embodiment, the reactants are provided simultaneously. The gel can then form while the active ingredient binds to the inorganic carrier. It is also possible to first form the gel and provided a solution thereof, then add the active ingredient and thereafter add the inorganic carrier. Depending on the application the most suitable sequence can be used.

In a preferred embodiment, the hybrid inducible release vehicle of the invention is prepared by first mixing the polymer and cross-linking agent and cross-linking the polymer to form the gel, thereafter adding the inorganic carrier is to a solution of the gel and thereafter adding the active ingredient.

The amount of active ingredient applied in the hybrid inducible release vehicle of the invention very much depends on the nature of the active ingredient, in particular the molecular mass of the active ingredient. The molecular mass of the active ingredient can vary widely and can for instance be at least 10 D, such as at least 50 D, at least 100 D, or at least 200 D. The upper limit of the molecular mass can be 300 kD, 280 kD or 240 kD. Further, the application for which the hybrid inducible release vehicle is to be used can be of great influence on the amount of active ingredient applied. For certain applications a very small amount of active ingredient will be preferred, while for other applications larger amounts can be advantageous. Typically, the amount of active ingredient in the vehicle can be at least 1 µg per g total dry weight solids, such as at least 5 µg per g total dry weight solids or at least 10 µg per g total dry weight solids. The amount of active ingredient in the vehicle can be as high as 10 mg per g total dry weight solids, such as 8 mg per g total dry weight solids, preferably 2 mg per g total dry weight solids.

An advantage of the present invention is that the active substance will only be released at the location where micro-organisms or specific enzyme mixtures are present and active. This means that, in the absence of *e*.*g*. micro-organisms or active eukaryotic cells, no migration of the antimicrobial substance to the outside will occur, and also that, in the presence of micro-organisms to be controlled, the amount of released antimicrobial substance will be limited to a minimum.

A further example is the release of medicines in the intestines where the vehicles can be decomposed by the intestinal enzymes or flora present and thus effect the release of an active substance. For this use, any therapeutically active ingredient can be used and the invention is not limited to antimicrobial agents. Preferably, those therapeutically active ingredients are used that run the risk of being decomposed in the mouth, esophagus or stomach. Thus, the vehicles of the invention can also be used for passage through the stomach of an acid- or protease-labile medicine or nutraceutical in the active form.

In addition, a vehicle comprising an antimicrobial active ingredient according to the invention can also very well be used in an anti-acne gel. Here again, the advantage compared to the known anti-acne agents is that the antimicrobial active ingredient is only released at the moment and at the location where the micro-organisms are present. This prevents undesired exposure of the skin to the antimicrobial agent.

In addition to use in anti-acne agents, the vehicle comprising the antimicrobial active ingredient according to the invention can also be used in other cosmetics. This is because it is known that cosmetics applied on the skin (*e*.*g*. creams, lotions, powders, and the like) are a food source for micro-organisms. So, infections of micro-organisms which use these applied cosmetics as a food source can be prevented by the invention. Thus, the invention also makes it possible for antimicrobial agents used in the current cosmetics (*e*.*g*. alcohol or alcohol derivates) to be left out of the cosmetics composition. This is especially advantageous because these agents often cause irritation of the skin. This skin irritation is absent if the vehicle with antimicrobial active ingredient according to the invention is used.

Another application is the use of a vehicle comprising an antimicrobial active ingredient according to the invention in dressing means, such as dressings for wounds, but also sanitary dressings. In wound healing, control of micro-organisms is a prerequisite and a dressing according to the invention contributes to the antimicrobial active ingredient being released only at locations where this is needed, and needless exposure of wound tissue to antimicrobial agents being prevented.

Other uses of antimicrobials packaged in a vehicle according to the invention which can be decomposed by micro-organisms are possible. Such an antimicrobial substance, in particular a fungicidal substance, can very well be used in fungicidal or anti-fouling paints. The advantage compared to conventional fungicidal or anti-fouling paints is that the paints according to the invention remain active much longer, since the antimicrobial (fungicidal, anti-fouling) substance is only released when there is reason to.

In addition, a coating comprising vehicles with an antimicrobial substance according to the invention can very well be used in vulnerable systems. In this context, vulnerable systems are systems (materials, humid environments) susceptible to infection by micro-organisms, such as (the cut stems of) cut flowers, plant roots, nutrient media of rock wool or other material, etc. Coating this type of materials using a coating according to the invention does not hinder the functions (*e*.*g*. water or nutrient intake) of the materials, but still provides a sufficient protection against micro-organisms.

Further, a coating according to the invention could also very well be used on surfaces which often come into contact with foods and can, in this manner, be a source of contamination. Examples of these are chopping boards for cutting meat, vegetables and the like, work tops or other surfaces on which foods are prepared or put aside, conveyor belts in industrial food preparation and processing, and storage means (racks, crates and the like) where foods are stored without protection. To guarantee sufficient antimicrobial capacity, the coatings have to be applied again after a certain period of time. To determine this moment, the coating can simply be tested by applying a micro-organism thereon on purpose and determining whether the coating still contains sufficient vehicles with antimicrobial agent to stop the growth of the micro-organism.

Coatings according to the invention can also be used to coat seeds for protection against attack by micro-organisms or to coat air ventilation filters. Seeds are often provided with coatings to provide nutrients for the sprouting seedlings in the first days after sprouting. This, however, is also a period when the seedlings are very vulnerable to infection by micro-organisms. A coating which comprises vehicles according to the invention which are degradable by said micro-organisms upon which degradation an antimicrobial active component would be released would protect seedlings against such infection. It is also possible to apply seed coatings which are degradable by amylase, which is an enzyme which is produced by the sprouts themselves. In this way the antimicrobial substance (or any other active component, such as a deterrent) will be released at the moment that the seeds are sprouting and will thus protect the fresh sprouts.

Coatings on air ventilation filters are known to collect a vast amount of micro-organisms. Sometimes the environmental conditions in or on such a filter are favourable for the generation of colonies of such micro-organisms, which, in turn, will clot the filter and make the filter inoperable. It will be clear that a coating with vehicles according to the invention, wherein such vehicles comprise an antimicrobial active ingredient, will prevent or inhibit formation of micro-organism colonies and thus increase the lifetime of such an air ventilation filter.

Vehicles according to the invention can also be used for addition to edible substances. Preferably, the vehicles will be degradable by amylase, which is abundant in the mouth due to the presence of saliva. Such vehicles could contain active components which would be useful for dental applications, such as fluoride compositions and/or anti-caries compounds. Examples of such compounds are sodium fluoride or fluoride complexing agents.

In another preferred embodiment of the invention such vehicles would comprise flavouring compounds, such as would be normally present in food or in dental care compounds. When used in foodstuffs, the application of the vehicles of the invention would provide a new taste sensation, wherein the flavouring compound is only released when the vehicles come into contact with the amylase in saliva. Thus, use of the vehicles in such a way could cause a slow change of taste of a foodstuff in the mouth.

In another preferred embodiment of the invention such vehicles would comprise active components such as iron or bitter tasting drugs or nutraceuticals. When used in foodstuffs, the application of the vehicles of the invention results in masking of the undesired flavour. Use of the vehicles in such a way does not result in release of the active component in the mouth, but release can be triggered by the low pH in the stomach or by enzymes other than amylase in the stomach or small or large intestine.

### Examples

### Synthesis of Mg-Al-CO₃ layered double hydroxide (LDH) at pH 7.86

The synthesis method described by Cantrell *et al*. in *Appl. Catal*., A 2005, *287*(2), 183-190 is applied. Chemicals were used as received. Mg(NO₃)_{2·}6H₂O, Al(NO₃)₃·9H₂O, (NH₄)₂CO₃, and NH₄OH were obtained from Fluka, Acros, and J.T. Baker. The synthesis was default to the assumption of a molar ratio of Mg to Al of 2:1.

### Example 1 Synthesis of MgAl LDH (Mg₂Al(OH)₆(CO)₃)_{0.5} at 65 °C

A solution of 51.45 g of Mg(NO₃)₂·6H₂O and 37.56 g of Al(NO₃)₃·9H₂O in 201.71 g of demineralised water was added dropwise during 1 hour to 200 ml of demineralised water at 65°C while stirring, simultaneously with 38.7 g of (NH₄)₂CO₃ in 200.37 g of demineralised water. The pH was kept constant at 7.86 with a 35 % NH₄OH solution using a pH-stat. After the addition the solution was kept at 65 °C for 3 more hours, while stirring. Subsequently, the solution was filtered and washed to pH 7 and dried at 118 °C for 18 hours.

### Example 2 Synthesis of MgAl LDH (Mg₂Al(OH)₆(CO)₃)_{0.5} at 55 °C

150 ml of a solution of 128.28 g of Mg(NO₃)₂·6H₂O and 94 g of Al(NO₃)₃·9H₂O in 500 g of demineralised water was added dropwise during 1 hour to 75 ml of demineralised water at 55 °C while stirring, simultaneously with 150 ml of a solution of 96.20 g of (NH₄)₂CO₃ in 500 g of demineralised water. The pH was kept constant at 7.86 with a 35 % NH₄OH solution using a pH-stat. After the addition the solution was kept at 65°C for 3 more hours, while stirring. Subsequently, the solution was filtered and washed to pH 7 and dried at 118°C for 18 hours.

### Example 3 Synthesis of gel

In 1 l of H₂O 14 g of NaOH and 200 g of a starch, in which 30 % of the glucose units are oxidised on the primary hydroxyl group, were solved. Subsequently, 0.65 g of NaBH₄ was added after which the mixture was stirred for 5 minutes. Then, 50 ml of glycerol diglycidyl ether cross-linker (obtained from Sigma/Aldrich) was added while stirring. After half an hour of stirring, the slightly viscous solution was placed at 40 °C overnight. This resulted in a firm (hard brittle) light yellow gel which contained large gas bubbles. The gel was pressed through a sieve (1 mm²) and was suspended four times in 2 1 of H₂O and precipitated with 51 of ethanol. Thereafter, the gel was washed three times with ethanol, three times with acetone and then dried to air. This yielded 248.3 g of dry powder with a free swelling in H₂O of 15.2 g/g.

### Example 4 Synthesis of gel

In 1 l of H₂O 14 g of NaOH and 200 g of Paselli SA2 (a starch product obtained from Avebe) was dissolved. Subsequently, 0.5 g of NaBH₄ was added after which the mixture was stirred for 5 minutes. Then, 50 ml of glycerol diglycidyl ether was added while stirring. After half an hour of stirring, the hardly viscous solution was placed at 40 °C overnight. This resulted in a firm (brittle) brown gel which contained large gas bubbles. The gel was pressed through a sieve (1 mm²) after which the yellow mass was suspended four times in 2 l of H₂O and precipitated with 5 l of ethanol. Thereafter, the gel was washed three times with ethanol, three times with acetone and then dried to air. This yielded 233.9 g of dry powder with a free swelling in H₂O of 7.74 g/g.

### Example 5 Synthesis of gel with MgAl LDH

A suspension of 12 g of the dry powder of Example 3 in 200 ml of H₂O was brought to and kept at 65 °C. While stirring, a solution of 51.42 g of Mg(NO₃)₂·6H₂O and 37.55 g of Al(NO₃)₃·9H₂O in 200.02 g of H₂O and a solution of 38.21 g of (NH₄)₂CO₃ in 200.16 g of H₂O were added dropwise at approximately equal speed during 0.75 hours. It was attempted to keep the pH between 7.6 and 8 with 35 % ammonia. A viscous gel was obtained. The gel was suspended in H₂O to a volume of 3.4 l. The volume of the gel in the water only decreased slowly (3.1 l after half an hour, 2.5 l after one hour, and 2.3 l after one and a half hour). Subsequently, 1 l of H₂O was removed by decanting. The volume was then increased to 7 l with H₂O. After 15 minutes 2 l of H₂O was removed by decanting. After replenishing with H₂O, followed by stirring and settling, 3 l of H₂O was decanted after 15 minutes. Subsequently, the volume was replenished to 7 l with H₂O and after 30 minutes 3.5 was decanted. Again the volume was replenished to 7 with H₂O and after 45 minutes 4 l was decanted. Then the volume was replenished to 5 l with H₂O and with acetone to a total volume of 7 l. After 15 minutes 2 l was removed by decanting, after which the volume was replenished to 7 l with acetone. After 20 minutes in a acetone/ice bath of -20 °C 2 l was decanted after which the volume was replenished to 7 l with acetone. After twice decanting 1 l and replenishing to 7 l with acetone the suspension was kept overnight. Then 5 l was decanted after which the volume was replenished to 7 l with acetone. After 30 minutes 3.4 l was decanted. The precipitate was then isolated on a glass filter (pore size 3) and washed 5 times with acetone and dried in warm air. This resulted in 31.96 g of powder which was further dried in a stove at 110 °C under nitrogen. This yielded 28.75 g of powder with a free swelling in water of 5.50 g/g.

### Example 6 Synthesis of gel with MgAl LDH

Similar to Example 5, but using 16 g of the dry powder of Example 4. After 3 hours of stirring at 65 °C the reaction mixture was only slightly viscous. After cooling to room temperature the reaction mixture was replenished to 5 l with H₂O. After 1 hour 4.2 l of H₂O was removed by decanting. The volume was replenished to 51 with H₂O after which 4.2 l was decanted. After replenishment to 51 the suspension was kept overnight at room temperature. Then 4.2 l was decanted and the volume replenished to 5 l with H₂O. Then 4.2 l was decanted after which the volume was replenished to 5 l with acetone. After decanting the precipitate was washed 4 times with acetone and dried in warm air. This yielded 37.55 g of powder that was further dried in a stove at 110 °C to a mass of 33.96 g.

### Example 7 Exchange of MgAl LDH with indigo carmine in air

0.18 g of the MgAl LDH of Example 1 was added to 25 g of H₂O; while stirring. Then, 6 ml of 4 M HCl was added dropwise while stirring. 4 ml of 35 % NH₄OH and 4 ml of a 1 % indigo carmine solution were simultaneously added dropwise to the resulting clear solution while stirring. The resulting white dispersion was filtered and washed after which it was freeze dried.

### Example 8 Exchange of gel with MgAl LDH with indigo carmine in air

1 g of the powder of Example 5 was added to 200 g of H₂O while stirring. Then 6 ml of 4 M HCl was added dropwise. Simultaneously 2 ml of 35 % NH₄OH and 2 ml of 1 % indigo carmine solution were added dropwise to the resulting clear solution while stirring. The resulting white dispersion was filtered and washed after which it was freeze dried.

### Example 9 Exchange of gel with MgAl LDH with indigo carmine in air

0.53 g of the powder of Example 6 was added to 100 g of H₂O while stirring. Then 6 ml of 4 M HCl was added dropwise. Simultaneously 2 ml of 35 % NH₄OH and 1 ml of 1 % indigo carmine solution were added dropwise to the resulting clear solution while stirring. The resulting white dispersion was filtered and washed after which it was freeze dried.

### Example 10 Exchange of MgAl LDH with indigo carmine under nitrogen

0.53 g of the MgAl LDH of Example 1 was added to 20 g of H₂O, while stirring. Then, while stirring 6 ml of 4 M HCl and 2 ml of a 1 % indigo carmine solution were added. The mixture is brought under vacuum and then held in nitrogen atmosphere. To the resulting clear solution 2 ml of 35 % NH₄OH is added dropwise while stirring. The resulting white dispersion is filtered after one hour of stirring and washed and dried at 60 °C.

The Examples were evaluated using thermogravimetric analysis. The results are shown in Table 1. The TGA curves of Examples 5 and 6 clearly show an exothermal peak around 300 °C (291.29 °C and 327.80 °C) and at 524 °C that lack at the pure LDH of Examples 1 and 2. These peaks originate from the starch. The ratio starch/LDH is 1:1 (w/w) for Example 5 and 1:1.7 (w/w) for Example 6.

**Table 1**

| | % decrease to 200 °C | % decrease to 1 000 °C | % residue |
|---|---|---|---|
| Example 1 | 10.64 | 33.45 | 55.91 |
| Example 2 | 16.83 | 26.43 | 56.74 |
| Example 5 | 6.15 | 62.78 (29.33 % org.) | 31.07 |
| Example 6 | 6.15 | 55.89 (22.44 % org.) | 37.96 |

## Claims

1. Hybrid inducible release vehicle for releasing an active ingredient, comprising a gel, which gel comprises a cross-linked carbohydrate or protein polymer and an inorganic carrier, and at least one active ingredient bound to said inorganic carrier, wherein the release of the active ingredient is triggered by contact of the vehicle with an external stimulus.

2. Hybrid inducible release vehicle according to claim 1, wherein the inorganic carrier is an inorganic particle selected from the group consisting of smectites, layered double hydroxides, Ag, Au, Fe₂O₃, MgCl₂, AlCl₃, ZnCl₂, SiO₂ TiO₂, ZnO, and MgO.

3. Hybrid inducible release vehicle according to claim 2, wherein the average particle size of the inorganic particles is 2-1 000 nm, preferably 5-500 nm, more preferably 10-100 nm, as measured by transmission electron microscopy.

4. Hybrid inducible release vehicle according to claim 2 or 3, wherein the aspect ratio of the inorganic particles is in the range of 1-500.

5. Hybrid inducible release vehicle according to any one of the preceding claims, wherein the polymer is a biopolymer selected from the group consisting of starch, cellulose, pectin, gelatine, a gum and derivatives and/or mixtures thereof.

6. Hybrid inducible release vehicle according to any one of the preceding claims, wherein the inorganic carrier has antimicrobial properties.

7. Hybrid inducible release vehicle according to any one of the preceding claims, wherein the at least one active ingredient is neutral and/or hydrophobic.

8. Hybrid inducible release vehicle according to any one of the preceding claims, wherein the external stimulus is an enzyme which is able to degrade the polymer.

9. Hybrid inducible release vehicle according to any one of the preceding claims, wherein the release of the at least one active ingredient is induced by electrostatic interaction, for example caused by a change in pH.

10. Hybrid inducible release vehicle according to any one of the preceding claims, wherein the binding of said at least one active ingredient to the inorganic carrier comprises one or more selected from the group consisting of electrostatic interaction, hydrogen bonding, metal-ligand interaction, and adsorption.

11. Hybrid inducible release vehicle according to any one of the preceding claims, wherein the at least one active ingredient is one selected from the group consisting of an anti-acne agent, a deodorant, a fungicide, an antifouling component, an antimicrobial compound, a cytostatic agent, a flavouring agent, a drug, nutrients for horticulture, flame retardants, and food additives such as peptides.

12. Method for making a hybrid inducible release vehicle according to any one of the preceding claims comprising
- providing a mixture of the polymer and a cross-linking agent;
- cross-linking the polymer thereby forming a gel;
- adding the inorganic carrier;
- adding the at least one active ingredient.

13. Method according to claim 12, wherein the inorganic carrier is added after the gel is formed.

14. Method according to claim 12 or 13, wherein the gel is loaded with the at least one active ingredient by extraction.

15. Use of a hybrid inducible release vehicle according to any one of claims 1-11 for masking off-flavour tasting compounds, such as bitter tasting medicine or nutraceuticals.

16. Use of a hybrid inducible release vehicle according to any one of claims 1-11 for providing a change of taste of a foodstuff in the mouth.

17. Use of a hybrid inducible release vehicle according to any one of claims 1-11 in edible compositions for releasing for dental protective compounds in the mouth, such as fluoride compositions and/or anti-caries compounds.

18. Foodstuff comprising a hybrid inducible release vehicle according to any one of claim 1-11.
